# EUROPEAN PATENT APPLICATION

(11) **EP 3 184 060 A1**
(43) Date of publication of application: **28.06.2017**
(21) Application number: 15202339.6
(22) Date of filing: 23.12.2015
(51) Int. Cl.: A61B 17/34, A61B 17/00, A61M 39/02

(54) **DEVICE FOR INTERVENTIONS IN THE ABDOMINOPELVIC CAVITY**

(71) Applicant: Region Syddanmark, 7100 Vejle (DK)
(72) Inventor: Zogovic, Sergej, 6400 Sønderborg (DK)
(74) Representative: Plougmann Vingtoft a/s

(57) **Abstract**

A surgical port for inspecting a body cavity of a patient, such as the abdominopelvic cavity, following surgery of the patient. A flexible head with a silicone base part is connected with a working lumen trough a longitudinal tube arranged for surgical inspection through an incision in the body cavity of the patient. An inflatable ballon for fixation of the surgical port in the incision, circumscribes a distal part of the tube, and a first input channel in the flexible head allows gas contact to the ballon for providing inflation via a separate channel arranged along the tube forming the working lumen. A second input channel in the flexible head is in contact with the working lumen to allow inflation of CO2 in the body cavity prior to inspection through the woring lumen.

## Description

### FIELD OF THE INVENTION

The present invention relates to the field of interventional medical devices. More specifically for enabling access to the abdominopelvic cavity for diagnostic and/or therapeutic purposes.

### BACKGROUND OF THE INVENTION

Pathological conditions in the abdominopelvic cavity and accompanying surgery are associated with the risk of major complications such as: bleeding, infection, local deficiency of blood supply to internal organs and anastomosis leakage. The surgical connection of separate tubular hollow organs to form a continuous channel, as between two parts of the intestine is referred to as the anastomosis. Diagnostic of these serious conditions is currently relaying on imagining diagnostic techniques, such as CT scanning, ultrasound scanning or monitoring clinical symptoms and signs. This approach has its limitations with a delayed diagnosis, prolonged hospitalization and increased mortality as a consequence.

Laparoscopic surgery also known as minimal invasive surgery is modern surgical technique, where surgical procedures are performed trough small incisions in the abdominal wall. An inert gas, e.g. carbon dioxide (CO₂), is insufflated in the abdomen to provide a working space and enable visualization of internal organs. Access ports are used to enable insertion of a video camera and several, different, thin instruments into abdominal cavity, during procedures. Access ports to abdominal cavity in laparoscopic surgery are known as trocars. A trocar consists of an obturator, a cannula or sleeve and a seal. Obturators are made of metal or plastic, with sharp or non-bladed tip. They are used to facilitate insertion of the trocar through the abdominal wall. After trocar insertion, obturator is removed leaving the cannula sleeve in place in the abdominal wall. The sleeves are basically tubes made of metal or plastic with smooth or threaded walls, which enables insertion of a laparoscope or other surgical instruments into abdominal cavity. The important functional characteristic of the trocar is its ability to provide a sealed instrumentation through the sleeve, minimizing gas and/or fluid leakage during passage of the instruments. This is achieved by different types, usually silicone made, valves are placed at the trocar housing in the upper part of the sleeve.

Multiple trocars are used during a surgical procedure to provide an access for both laparoscope and surgical instruments. To address this issue a multi-lumen, single port devices were developed. Examples can be seen in e.g. EP2138106A2, US20100286484, and US20130184646. The key functional part of those devices is as sealing assemblies incorporated in flexible sleeve housing embodying multiple working channels. Both trocars and multi-lumen access port devices are used as a port to the abdominal cavity only during surgical procedure and are removed at the end of operation.

### SUMMARY OF THE INVENTION

Thus, according to the above description, it may be seen as an object of the present invention to provide a medical device which enables prolonged (up to such as 10 days, 14 days or even longer, such as more than 20 days), percutaneous access to abdominal and pelvic cavities after surgical procedure. Especially, it may be seen as a purpose of the invention to provide a medical device which enables inserting of a flexible visualization device (endoscope) into abdominal and pelvic cavities after surgical procedure for the purposes of observation as well as treatment.

In a first aspect, the invention provides a surgical port for inspecting a body cavity of a patient, such as the abdominopelvic cavity, following surgery of the patient, the surgical port comprising:
- a flexible head in connection with a through-going tubular part having a sealing mechanism allowing surgical inspection through an incision in the body cavity of the patient, the flexible head having a base part facing the skin of the patient made of an elastomeric material, preferably made of silicone or at least with a silicone coating,
- an inflatable balloon, such as formed by a thin foil, fastening mechanism circumscribing at least a part of the through-going tubular part, and the inflatable balloon being inflatable so as to secure fixation of the surgical port in said incision in the body cavity from inside,
- a first input channel arranged to provide fluid, e.g. a sterile gas, contact between the flexible head and the inflatable balloon fastening mechanism, such as to allow application of preferably sterile air to inflate the inflatable balloon, and
- a second input channel arranged for introducing a fluid, such as an inert gas e.g. CO₂ or helium or a combination of inert gases, preferably CO₂, into the body cavity prior to inspection through the through-going tubular part.

Such surgical port allows medical examination by enabling percutaneous access to abdominal and pelvic cavities after surgical procedure for the purpose of observation as well as treatment. The inflatable ballon enables the surgical port to several days, e.g. up to 10 days or even more, such as at least 14 days, or 20 days or more, so as to enable medical observations and treatments over a period of time after a surgery. Hereby, various medical conditions can be observed, e.g. using a sterile endoscope in combination with CO₂ inflation of the abdominal cavity, thus avoiding scannings which may not provide as precise information as using e.g. an endoscope. At the same time, if a critical medical condition is observed, treatment can be immediately applied via the surgical port without any further delay involved in connection with providing an incision etc. E.g. the surgical port allows treatments such as: 1) visually guided drainage of the abdominal and pelvic abscesses, 2) taking samples of body fluid and tissue from abdominal cavity, and 3) postoperative decompression of stomach and duodenum in duodenal perforation.

The possibility to periodically provide medical inspections of a patient through the surgical port for a long period of time after a surgery is important for some medical conditions, so as to observe the evolvement in the medical condition of the patient following the surgery. Especially this is important in medical cases where there is a known high risk that a worsening can occur. Here the surgical port can be inserted just after surgery, thus eliminating the need for any further incision of the patient at a later state, if a worsening requires endoscopic inspection or if an incision would be otherwise required for treatment in the body cavity.

Thus, both medical examination or observation, as well as treatment can be performed post operatively via such surgical port without the need for making any further postoperative incision.

In the following, preferred embodiments and preferred features are described.

The first input channel preferably has an inlet opening positioned on a side of the flexible head. The second input channel preferably has an inlet opening positioned on a side of the flexible head. Especially, the first input channel has an opening positioned on one side of the flexible head, and wherein the second input channel has an inlet opening positioned on an opposite side of the flexible head. This can allow easy access for applying gas for inflation of the fastening ballon from one side, and applying e.g. CO₂ for inflation of the body cavity from the opposite side, such that tubes involved for these two activities do not interfere. Instead it may be preferred that the first and second input channels are arranged with their inlet openings at another angle, seen in top view, rather than 180° (i.e. opposite), e.g. forming an angle of 30°-150°, e.g. an angle of 45°-135°, such as 70°-110°, e.g. 90° or about 90°. E.g. the first and second input channels are arranged with their inlet openings in the same horizontal plane, however they may also be preferred to be arranged on top of each other, i.e. in different horizontal planes, e.g. on the same side of the flexible head, seen in top view, or providing different angles, seen in top view, as just described.

Preferably, an opening to said through-going tubular part having a sealing mechanism is positioned on a top part of the flexible head, such as said opening being positioned at a central portion of the flexible head. Especially, said opening may face upwards, so as to allow access to said through-going tubular part with an elongate instrument, when positioned on the patient, e.g. an endoscope. Specifically, a stopper arranged to enter said opening to said through-going tubular part, wherein the stopper is formed as a monolithic part of the flexible head, such as a monolithic part of the base part of the flexible head.

The base part of the flexible head may be formed monolithically with at least a part of a tube forming the through-going tubular part, such as in a silicone material.

Preferably, a valve, e.g. comprising a luer check valve, is arranged at an externally accessible opening to the first input channel, wherein the valve is arranged inside the flexible head.

Preferably, a valve, e.g. a luer check valve, is arranged at an externally accessible opening to the second input channel, wherein the valve is arranged inside the flexible head.

Preferably, said sealing mechanism comprises a duckbill valve. Preferably, the duckbill valve sized for insertion of a medical instrument.

A tube forming the through-going tubular part may have a first lumen for allowing surgical inspection, and a second lumen connected to the second input channel. The first and second lumens may extend longitudinally in parallel at least along a major portion of the tube.

A lumen of the through-going tubular part, and the sealing mechanism may be coated with a surface active antimicrobial coating.

The surgical port is preferably arranged for remaining positioned through the incision in the body cavity of the patient for a period of at least 2 days, such as at least 3 days, such as at least 4 days, such as at least 5 days, e.g. at least 10 days, e.g. at least 14 days, e.g. at least 20 days, e.g. longer than 20 days, so as to allow abdominopelvic cavity inspecting throughout the period without removing the surgical port. Thus, the surgical port has a surface in contact with the body of the patient when inserted, wherein this surface is designed to be in contact with the body of the patient for at least 2 days, e.g. up to 10 days, e.g. up to 14 days, e.g. up to 20 days, e.g. longer than 20 days, without causing any severe medical risk.

The flexible head may especially have a circular shaped top part, seen in a top view, and wherein an opening to the through-going tubular part is positioned in a central part of the circular shaped top part.

The first input channel is most preferably suited for applying a gas, rather than a liquid, preferably a sterile gas, e.g. sterile air, for inflation of the inflatable balloon. The second input channel is most preferably suited for applying a gas rather than a liquid, preferably an inert gas, such as CO₂.

In a second aspect, the invention provides a kit of a plurality of surgical ports according to the first aspect, wherein said plurality of surgical ports have different lengths of their through-going tubular parts. E.g. lengths of the through-going tubular parts measured between the lower side of the base part of the flexible head and the distal tip of the through-going tubular part. It is to be understood that the distance between the lower side of the base part of the flexible head and an upper fastening point of the inflatable balloon is preferably also different. Further, the kit may comprise different sizes of inflatable balloons, e.g. inflatable balloons with different volumes.

Such kit of surgical ports is advantageous for fitting to different patients, i.e. for patients with different thickness of cutaneous and subcutaneous tissue of the patient, e.g. of their abdominal wall. A suitable one of the plurality of surgical ports of the kit can be selected after measuring the thicknes of the abdominal wall of the patient. E.g. the kit may comprise 2, 3, 4, 5, 6, or more surgical ports with through-going tubular parts of different lengths.

In a third aspect, the invention provides a method for inserting a surgical port according to the first aspect through an incision in the body cavity of a patient, such as in the abdominopelvic cavity of the patient.

The method preferably comprises, after the insertion, inflating the inflatable balloon by applying a fluid, e.g. a gas, through the first input channel in the surgical port.

The method may further comprise inserting a medical examination instrument through the through-going tubular part in the surgical port, and further comprising inflating the abdominopelvic cavity by applying an inert gas, e.g. CO₂, via the second input channel.

The method may further comprise inserting a treatment instrument, such as a drainage device or a surgical device, via the through-going tubular part in the surgical port.

The method preferably comprises letting the surgical port remain in place in the patient with the ballone inflated, and removing the surgical port after a period of at least 2 days after the insertion, such as after a period of at least 5-10 days after the insertion, such as 5-10 days after the insertion, such as at least 14 days after the insertion, such as at least 20 days after the insertion.

It is appreciated that the same advantages and embodiments described for the first aspect apply as well for the second and third aspects. Further, it is appreciated that the described embodiments can be intermixed in any way between all the mentioned aspects.

### BRIEF DESCRIPTION OF THE FIGURES

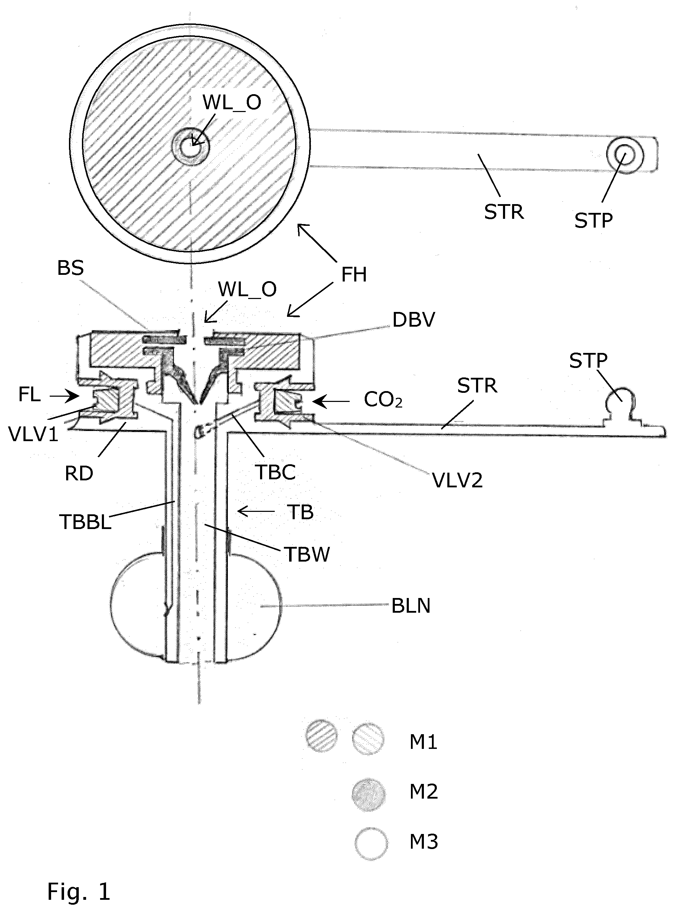

The invention will now be described in more detail with regard to the accompanying Fig. 1 which illustrates a top view and a section view of a preferred surgical port embodiment.

The figure illustrate specific ways of implementing the present invention and are not to be construed as being limiting to other possible embodiments falling within the scope of the attached claim set.

### DETAILED DESCRIPTION OF THE INVENTION

Fig. 1 shows a surgical port embodiment for inspecting a body cavity of a patient, specifically e.g. the abdominopelvic cavity, following surgery of the patient. The upper parf ot Fig. 1 shows a top view of the surgical port, while a side section view is seen below. Further below, an indication of the different materials can be seen next to their visual indication on the drawing: M1 indicates a plastic material, M2 indicates a rubber material, and M3 indicates a medical silicone material.

A flexible head FH, i.e. a top part of the surgical port, forms a proximal end of the surgical port, and it is connected with a tube TB which surrounds the through-going tubular part or working lumen TBW. A sealing mechanism, here shown as a duckbill valve DBV and an additional back-up seal BS, is designed to allow surgical inspection, e.g. by an endoscope, through the working lumen TBW through an incision in the body cavity of the patient.

The flexible head FH has a base part, or external retention disc RD, facing the skin of the patient when inserted. This base part or external retention disc RD is made of an elastomeric material, preferably it is made of silicone or at least with a silicone coating.

To enable the surgical port to remain fastened, after insertion, an inflatable balloon fastening mechanism BLN. Such balloon BLN may be formed by a thin foil. The balloon BLN circumscribes at least a part of the distal end of the tube TB forming the through-going tubular part or the working lumen. E.g. the inflatable ballon BLN may circumscribe the entire part of the tube TB. The inflatable balloon BLN is inflatable so as to secure fixation of the surgical port in said incision in the body cavity from inside, and it is connected via a first input channel FL arranged to provide gas contact between the flexible head FH and the inflatable balloon fastening mechanism BLN, so as to allow inflation from outside by a sterile gas, when the surgical port has been inserted.

A luer check valve VLV1 serves to keep the ballon BLN inflated after removing an inflation pipe or tube for supplying the gas to the first input channel FL. The inflatable balloon BLN is located at a distal end of the tube TB, functioning as internal bolster and is inflated through an inflation valve located on the base. The tube TB has a working lumen TBW and an inflation lumen TBBL, extending parallel longitudinally. The inflation lumen TBBL is arranged at an inclined angle ending with a non-return check valve VLV1, e.g. a luer check valve, incorporated in a base part of the flexible head FH.

A second input channel CO₂ is arranged for introducing a gas, such as an inert gas, preferably CO₂, into the body cavity prior to inspection through the through-going tubular part or working lumen TBW in the tube TB. This input channel CO₂ is connected to the working lumen TBW of the tube TB via an approximately 2 mm wide connection channel TBC in the flexible head FH, and a non-return valve VLV2, e.g. a luer check valve, is positioned in the base part of the flexible head FH. This allows insufflation of CO₂ intraperitoneally through the valve VLV2, providing a space for the intraperitoneal examination. Continuous, controlled insufflation of CO₂ during examination is providing sustained intraperitoneal pressure. It is to be understood that careful design of the connection channel TBC and its connection to the working lumen TBW can help to medical instrument to prevent problems with the sticking to the duckbill valve DWW during insertion, since the application of CO₂ near the duckbill valve DBW can help to reduce friction. Thus, careful design of this connection channel TBC for CO₂ applciation may allow the use of a duckbill valve DBW with smaller dimensions and still allow insertion of a medical instrument of a given size without sticking problems.

As seen, the input channel FL for applying gas for inflating the fastening ballon BLN is arranged opposite to the input channel CO₂ for applying CO₂.

The body of the surgical port is made of medical grade material M3, preferably silicone. Lumen of the working channel TBW, duckbill valve DBV and sealing valve BS is preferably coated with a surface active antimicrobial coating.

The tubular part TB of the surgical port is inserted intraperitoneally either through a previous incision after laparoscopy or through a new skin incision and under visual guidance. The tubular part TB is mounted on an obturator and inserted through the incision into the abdominal or pelvic cavity. The balloon BLN is inflated by insufflating a sterile air through the non-return valve VLV1, embodied in the silicone basis of the flexible head FH. This non-return valve VLV1 is connected with the balloon BLN in the distal part of the tubular part TB with a thin channel TBBL embodied in the wall of the tubular part RB and running parallel with the larger working channel in the tube TB. After inflation of the balloon BLN, the obturator is removed. The inflated balloon provides internal bolster and in combination with the retention disc RD part of the device anchor the device against abdominal wall.

The duckbill valve DBV in the basis of the flexible head FH closes the lumen of the working channel in the tube TB preventing gas escaping from the peritoneopelvic cavity before the introduction or after withdrawal of endoscope into the lumen TBW of the working channel through the external opening WL_O on the basis of the surgical port. The sealing valve BS, DBV placed just below external opening WL_O of the working channel, provides additional sealing during instrumentation through the lumen TBW of the working channel, limiting CO2 leakage during examination. The device can be safely removed after balloon BLN is desufflated by removing air from the balloon BLN through a non-return valve VLV1 in the flexible head basis.

In the top view, it is seen that the flexible head FH has a circular shape with the working lumen opening WL_O placed in the centre, facing upwards, so as to allow easy insertion of an elongate instrument the working lumen TBW in the straight shaped tube TB. The base part or retention disc RD of the flexible head FH is monoliticitally formed with a strap STR having a stopper STP formed on a distal end. This stopper STP is shaped to match the opening WL_O of the working lumen TBW, so as to allow the stopper to be placed in the working lumen openining WL_O, thus forming yet another external sealing of the opening to the working lumen TBW of the TB, when not used for examination or treatment.

Due to a variable thickness of the abdominal wall in humans, a device with an appropriate length of the tubular part TB should be chosen after measuring the thickness of cutaneous and subcutaneous tissue of the patient.

The valve VLV1 connected to the balloon BLN and the valve VLV2 for CO₂ insufflation may e.g. be marked with a different color (blue and green) and a text "AIR" and "CO2" designating their usage.

To sum up: the invention provides a surgical port for inspecting a body cavity of a patient, such as the abdominopelvic cavity, following surgery of the patient. A flexible head with a silicone base part is connected with a working lumen trough a longitudinal tube arranged for surgical inspection through an incision in the body cavity of the patient. An inflatable ballon for fixation of the surgical port in the incision, circumscribes a distal part of the tube, and a first input channel in the flexible head allows gas contact to the ballon for providing inflation via a separate channel arranged along the tube forming the working lumen. A second input channel in the flexible head is in contact with the working lumen to allow inflation of CO2 in the body cavity prior to inspection through the woring lumen.

Although the present invention has been described in connection with the specified embodiments, it should not be construed as being in any way limited to the presented examples. The scope of the present invention is to be interpreted in the light of the accompanying claim set. In the context of the claims, the terms "including" or "includes" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. A surgical port for inspecting a body cavity of a patient, such as the abdominopelvic cavity, following surgery of the patient, the surgical port comprising:
- a flexible head (FH) in connection with a through-going channel (TBW, TB) having a sealing mechanism (BS, DBV) allowing surgical inspection through an incision in the body cavity of the patient, the flexible head (FH) having a base part (RD) facing the skin of the patient made of an elastomeric material, preferably silicone,
- an inflatable balloon (BLN), such as formed by a thin foil, fastening mechanism circumscribing at least a part of the through-going channel (TB), and the inflatable balloon (BLN) being inflatable so as to secure fixation of the surgical port in said incision in the body cavity from inside,
- a first input channel (FL) arranged to provide fluid, such as a sterile gas, contact between the flexible head (FH) and the inflatable balloon fastening mechanism (BLN), and
- a second input channel (CO₂) arranged for introducing a fluid, such as an inert gas, preferably for carbon dioxide, into the body cavity prior to inspection through the through-going channel (TB).

2. Surgical port according to claim 1, wherein the second input channel (CO₂) has an inlet opening positioned on a side of the flexible head (FH).

3. Surgical port according to claim 1 or 2, wherein the first input channel (FL) has an inlet opening positioned on a side of the flexible head (FH).

4. Surgical port according to claim 2, wherein the first input channel (FL) has an opening positioned on one side of the flexible head (FH), and wherein the second input channel (CO₂) has an inlet opening positioned on an opposite side of the flexible head (FH).

5. Surgical port according to any of the preceding claims, wherein an opening to said through-going channel (TB) having a sealing mechanism (BS, DBV) is positioned on a top part of the flexible head (FH), such as said opening (WL_O) being positioned at a central portion of the flexible head (FH).

6. Surgical port according to claim 5, wherein said opening (WL_O) faces upwards, so as to allow access to said through-going channel (TB) with an elongate instrument, when positioned on the patient.

7. Surgical port according to claim 5 or 6, comprising a stopper (STP) arranged to enter said opening (WO_L) to said through-going channel (TB), wherein the stopper (STP) is formed as a monolithic part of the flexible head (FH), such as a monolithic part of the base part (RD) of the flexible head (FH).

8. Surgical port according to any of the preceding claims, wherein the base part (RD) of the flexible head is formed monolithically with at least a part of a tube (TB) forming the through-going channel.

9. Surgical port according to any of the preceding claims, comprising a valve (VLV1), such as a luer check valve, arranged at an externally accessible opening to the first input channel (FL), wherein the valve (VLV1) is arranged inside the flexible head (FH).

10. Surgical port according to any of the preceding claims, comprising a valve (VLV2), such as a luer check valve, arranged at an externally accessible opening to the second input channel (CO₂), wherein the valve (VLV2) is arranged inside the flexible head (FH).

11. Surgical port according to any of the preceding claims, wherein said sealing mechanism comprises a duckbill valve (DBV) arranged for insertion of a medical instrument.

12. Surgical port according to any of the preceding claims, wherein a tube (TB) forming the through-going channel has a first lumen (TBW) for allowing surgical inspection, and a second lumen (TBBL) connected to the second input channel (FL).

13. Surgical port according to claim 12, where the first and second lumens (TBW, TBBL) extend longitudinally in parallel at least along a major portion of the tube (TB).

14. Surgical port according to any of the preceding claims, wherein a lumen (TBW) of the through-going channel, and the sealing mechanism (BS, DBV) is coated with a surface active antimicrobial coating.

15. Surgical port according to any of the preceding claims, being arranged for remaining positioned through the incision in the body cavity of the patient for a period of at least 2 days, such as at least 3 days, such as at least 4 days, such as at least 5 days, such as 5-10, days such as at least 14 days, such as at least 20 days, so as to allow abdominopelvic cavity inspecting throughout the period without removing the surgical port.
